# EUROPEAN PATENT APPLICATION

(11) **EP 4 537 761 A1**
(43) Date of publication of application: **16.04.2025**
(21) Application number: 22945915.1
(22) Date of filing: 31.08.2022
(51) Int. Cl.: A61B 7/04

(54) **MOBILE INFORMATION TERMINAL, INFORMATION PROCESSING SYSTEM, AND CONTROL METHOD AND PROGRAM FOR MOBILE INFORMATION TERMINAL**

(30) Priority: 07.06.2022 JP 2022092104
(71) Applicant: Suntory Holdings Limited, Osaka 530-8203 (JP)
(72) Inventor: KUTSUMI Yuka, Kyoto 619-0284 (JP); KANEGAWA Norimasa, Kyoto 6190284 (JP)
(74) Representative: Vossius & Partner Patentanwälte Rechtsanwälte mbB
(86) International application number: PCT/JP2022/032847
(87) International publication number: WO 2023/238419

(57) **Abstract**

When a conventional portable information terminal is used to record abdominal sounds, there is a problem in that it is difficult to acquire a high-quality recording result. It is possible to acquire a high-quality recording result of abdominal sounds by using a portable information terminal 6 that has two or more microphones and is used to record a user's abdominal sounds in a state where a sound collecting portion near one of the two or more microphones is in contact with the user's abdomen, the portable information terminal 6 including: a judgment unit 47 that judges whether or not a predetermined control condition related to recording of the abdominal sounds is satisfied; and a microphone control unit 48 that controls one or more microphones among the two or more microphones when the control condition is judged to be satisfied.

## Description

### Technical Field

The present invention relates to a portable information terminal having a plurality of built-in microphones, an information processing system, and a method for controlling a portable information terminal, and a program.

### Background Art

In recent years, users have become increasingly health conscious. Under such circumstances, various apparatuses and services are now available to support health and lifestyle habit information. For example, Patent Document 1, listed below, describes the configuration of a health management support system that is configured to acquire and record a user's health management information and output health management-related messages to the user based on the contents of the health management information.

The intestines (small intestine and large intestine) are known as one of the most important organs for digestion and absorption of nutrients and for symbiosis with gut bacteria. In recent years, there has been an increasing need for gut health, which is reflected in the term "gut health", for example.

Patent Document 2, listed below, describes an analyzing apparatus that evaluates gastrointestinal motility for the purpose of diagnosing intestinal diseases based on acoustic features extracted using machine learning techniques from sound recording data acquired by a non-contact microphone.

In addition, Non-Patent Document 1, listed below, describes the recording of gut sounds over a relatively long period of time using a low-cost, simple piezoelectric acoustic sensing device for use in the diagnosis of digestive diseases.

### Citation List

### Patent Documents

Patent Document 1: JP 2017-041035A
Patent Document 2: WO 2019/216320A1

### Non-Patent Document

Non-Patent Document 1: Du, X. et al. 2018 Bowel Sounds Identification and Migrating Motor Complex Detection with Low-Cost Piezoelectric Acoustic Sensor. Sensors. 18 (12), 4240

### Summary of Invention

### Technical Problem

If it becomes possible to record a user's abdominal sounds and acquire information that is useful for managing the user's health, it will be possible to contribute to the user's health management. In particular, if a user's abdominal sounds can be recorded using a widely used portable information terminal such as a smartphone, the user's health can be easily managed. However, when abdominal sounds are recorded using such a portable information terminal, there is a problem in that it is difficult to acquire a high-quality recording result.

Therefore, the present invention aims to provide a portable information terminal, an information processing system, and a method for controlling a portable information terminal, and a program that are capable of acquiring a high-quality recording result of abdominal sounds.

However, when a conventional portable information terminal is used to record abdominal sounds, there is a problem in that it is difficult to acquire a high-quality recording result.

### Solution to Problem

A portable information terminal according to a first aspect of the present invention is a portable information terminal that has two or more microphones and is used to record a user's abdominal sounds in a state where a sound collecting portion near one of the two or more microphones is in contact with the user's abdomen, the portable information terminal including: a judgment unit that judges whether or not a predetermined control condition related to recording of the abdominal sounds is satisfied; and a microphone control unit that controls one or more microphones among the two or more microphones when the control condition is judged to be satisfied.

With such a configuration, it is possible to control the microphones and acquire a high-quality recording result of abdominal sounds.

A portable information terminal according to a second aspect of the present invention is the portable information terminal according to the first aspect of the invention, wherein the microphone control unit performs on and off control on the one or more microphones when the control condition is judged to be satisfied.

With such a configuration, it is possible to turn on only the necessary microphones to acquire a recording result of abdominal sounds.

A portable information terminal according to a third aspect of the present invention is the portable information terminal according to the first or the second aspect of the invention, wherein the microphone control unit controls parameters related to operation of the one or more microphones when the control condition is judged to be satisfied.

With such a configuration, it is possible to operate the microphones so as to be able to acquire a high-quality recording result of abdominal sounds.

A portable information terminal according to a fourth aspect of the present invention is the portable information terminal according to any one of the first to third aspects of the invention, wherein the microphone control unit acquires an identifier identifying a model of the portable information terminal, and controls the one or more microphones in accordance with the acquired identifier.

With this configuration, it is possible to perform control so as to be able to record high-quality recording result of abdominal sounds for each model of portable information terminal.

A portable information terminal according to a fifth aspect of the present invention is the portable information terminal according to any one of the first to fourth aspects of the invention, wherein the predetermined control condition is that the portable information terminal enters a predetermined preparation state for recording the abdominal sounds.

With such a configuration, it is possible to control the microphones and acquire a high-quality recording result of abdominal sounds.

A portable information terminal according to a sixth aspect of the present invention is the portable information terminal according to any one of the first to fourth aspects of the invention, wherein the predetermined control condition is that sound information acquired through the recording satisfies a predetermined interruption condition during the recording of the abdominal sounds.

With such a configuration, it is possible to control the microphones so as to be able to acquire a high-quality recording result of abdominal sounds during the recording of the abdominal sounds.

An information processing system according to a seventh aspect of the present invention is an information processing system including: the portable information terminal according to any one of the first to sixth aspects of the invention; and an information processing apparatus capable of communicating with the portable information terminal, wherein the portable information terminal includes a terminal output unit that outputs sound information acquired by recording a user's abdominal sounds using the one or more microphones, and the information processing apparatus includes: a sound information acquisition unit that acquires the sound information; a gut score acquisition unit that acquires a gut score related to the user's gut condition, using input information containing the sound information acquired by the sound information acquisition unit and learning information prepared in advance; and a gut score output unit that outputs the gut score acquired by the gut score acquisition unit.

With such a configuration, it is possible to output an intestinal score related to the user's intestinal condition, using a high-quality recording result of abdominal sounds.

A method for controlling a portable information terminal according to an eight aspect of the present invention is a method for controlling a portable information terminal that has two or more microphones and is used to record a user's abdominal sounds in a state where a sound collecting portion near one of the two or more microphones is in contact with the user's abdomen, the method including: a judgment step of judging whether or not a predetermined control condition related to recording of the abdominal sounds is satisfied; and a microphone control step of controlling one or more microphones among the two or more microphones when the control condition is judged to be satisfied.

### Advantageous Effects of Invention

With the portable information terminal and so on according to the present invention, it is possible to acquire a high-quality recording result of abdominal sounds.

### Brief Description of Drawings

FIG. 1 is a diagram showing an overview of an information processing system according to one embodiment of the present invention.
FIG. 2 is a block diagram for an information processing apparatus according to the same.
FIG. 3 is a block diagram for a portable information terminal according to the same.
FIG. 4 is a perspective view showing the portable information terminal according to the same.
FIG. 5 is a diagram showing an example of a situation in which a user records abdominal sounds with the portable information terminal according to the same.
FIG. 6 is a flowchart showing examples of operations of the information processing apparatus according to the same.
FIG. 7 is a flowchart showing examples of operations of the information processing apparatus according to the same.
FIG. 8 is a diagram showing specific examples of screen transitions of the portable information terminal according to the same.
FIG. 9 is an overview diagram for a computer system according to the above embodiments.
FIG. 10 is a block diagram for the computer system according to the same.

### Description of Embodiments

Hereinafter, embodiments of a portable information terminal, an information processing system using the same, and so on will be described with reference to the drawings. Note that in the embodiments, constituent elements with the same reference signs perform similar operations, and therefore, repeated descriptions thereof may be omitted.

In the following description, the terms used are generally defined as follows. Note that the meanings of these terms should not always be interpreted as given here, but, for example, where they are described individually below, they should be interpreted in the light of that description.

Abdominal sounds refer to sounds emitted from a user's abdomen. Abdominal sounds can include, for example, gut sounds emitted from the intestines. Abdominal sounds may also include sounds caused by blood flow in the abdomen (for example, abdominal aortic sounds) and sounds emitted from organs such as the stomach.

Sound information refers to information acquired based on abdominal sounds. Sound information may be the recorded abdominal sound data itself, or may be data obtained by processing or editing the recorded data, or the like.

Lifestyle information is information regarding a user's living status. The living status may include various elements related to a user's actions, conditions, and so on. Lifestyle information may include, for example, excretion-related information regarding a user's excretion status, dietary information regarding a user's dietary status, situational activities related to lifestyle habits other than the dietary status (referring to actions and behaviors that the user repeatedly performs in their daily life), and so on. However, lifestyle information is not limited to the above examples, and may include information regarding other elements, or may include information regarding only some of these elements. Note that each piece of information regarding the elements of lifestyle information (excretion-related information, dietary information, activity status information, and so on) may include information regarding smaller elements.

Basic information is information regarding a user's morphology and characteristics. For example, basic information may include various information such as the user's height, weight, age, sex, body fat percentage, muscle mass, pulse rate, respiratory rate, and blood pressure. Basic information may also include the user's responses to a questionnaire, information generated based on the responses, information regarding the user's medical history, and so on. For example, responses to questionnaire items used to diagnose people with intestinal disorders can objectively represent the user's constitution. Basic information is, for example, information that is input in advance by the user, but this is not essential, and basic information may be, for example, information acquired by a wearable terminal or the like worn by the user, or information acquired from an external database or the like in which user information is recorded.

An identifier for an item is a character or code that uniquely identifies the item. An identifier is, for example, an ID, but there is no limitation on the type thereof as long as it is information that can identify the corresponding item. That is to say, an identifier may be the name of the item it indicates, or may be a combination of codes that uniquely correspond to the item.

Acquisition may include acquiring information input by a user or the like, or may include acquiring information stored in the apparatus itself or another apparatus (which may be information stored in advance or information generated by information processing performed in the apparatus). Acquiring information stored in another apparatus may include acquiring information stored in the other apparatus via an API or the like, or may include acquiring the contents of a document file (including the contents of a web page) provided by the other apparatus. It may also include acquiring information in a different format from the original information, such as acquiring information by performing optical character reading on an image.

In addition, so-called machine learning techniques may be used to acquire information. Machine learning techniques can be used in the following manner, for example. That is to say, a learning model (learning information) that receives a specific type of input information as input and outputs the type of output information that is desired to be acquired is formed using machine learning techniques. For example, two or more sets of input information and output information are prepared in advance, and the two or more sets of information are provided to a module for forming a machine learning model to form a learning model, and the learning model thus formed is accumulated in a storage unit. The learning model can also be called a classifier. Examples of machine learning techniques include deep learning, random forest, SVM, and so on, and there is no limitation. In addition, functions in various machine learning frameworks such as fastText, tinySVM, random forest, and TensorFlow, and various existing libraries, can be used for machine learning.

In addition, the learning model is not limited to one acquired by machine learning. The learning model may be, for example, a table indicating the correspondence between input vectors that are based on input information or the like and pieces of output information. In this case, the piece of output information corresponding to the feature vector based on input information may be acquired from the table, or a vector approximating the feature vector based on input information may be generated using two or more input vectors in the table and parameters that weight the input vectors, and the final output information may be acquired using the pieces of output information and parameters corresponding to the input vectors used in the generation. The learning model may be, for example, a function that represents the relationship between input vectors that are based on input information or the like, and pieces of information for generating output information. In this case, for example, information corresponding to a feature vector that is based on input information may be obtained using the function, and output information may be obtained using the obtained information.

The "output" of information is a concept that encompasses displaying on a display screen, projection using a projector, printing by a printer, the output of a sound, transmission to an external apparatus, accumulation on a recording medium, delivery of a processing result to another processing apparatus or another program, and the like. Specifically, this concept includes, for example, enabling the information to be displayed on a web page, sending the information as an e-mail or the like, outputting information for printing, and so on.

The "acceptance" of information is a concept that includes, for example, acceptance of information input from an input device such as a keyboard, a mouse, or a touch panel, reception of information transmitted from another apparatus or the like via a wired or wireless communication network, or acceptance of information read out from a recording medium such as an optical disk, a magnetic disk, or a semiconductor memory.

### Embodiments

In the present embodiment, a smartphone having two or more microphones is used as the portable information terminal. The portable information terminal is used to record gut sounds by pressing it against the abdomen. The portable information terminal controls one or more microphones when a predetermined control condition is satisfied. Hereinafter, an information processing system 1 formed using such a portable information terminal will be described.

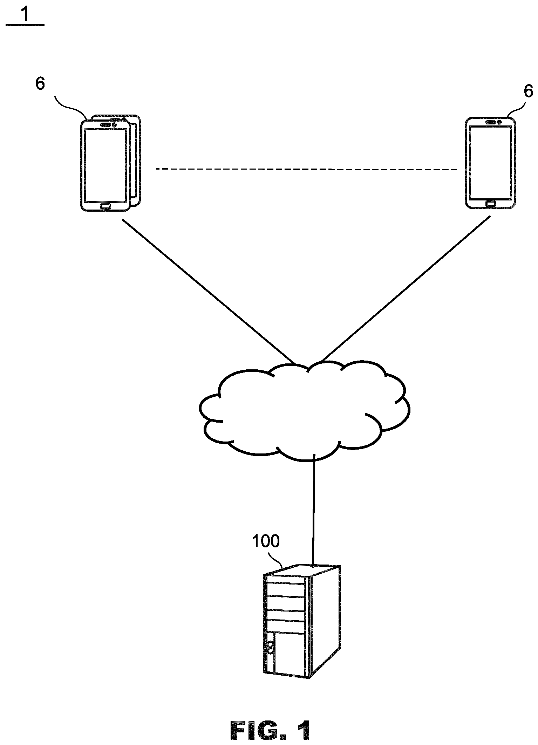

FIG. 1 is a diagram showing an overview of the information processing system 1 according to one embodiment of the present invention.

In the present embodiment, the information processing system 1 includes an information processing apparatus 100 and a portable information terminal 6. The information processing apparatus 100 and the portable information terminal 6 are capable of communicating with each other via a network such as a local area network or the Internet. Note that the configuration of the information processing system 1 is not limited to this example. There is no limitation on the number of apparatuses included in the information processing system 1, and other apparatuses may also be included in the information processing system 1.

A user of the information processing system 1 can use the information processing system 1 by using the portable information terminal 6. The portable information terminal 6 may also be referred to as a terminal apparatus. In the present embodiment, the portable information terminal 6 is a so-called smartphone. Note that a terminal that can be used as the portable information terminal 6 is not limited to such a smartphone. For example, a portable information terminal that is a personal computer (PC) such as a laptop computer may be used, or other apparatuses such as a tablettype information terminal apparatus may be used. In the following example, it is assumed that a smartphone is used as the portable information terminal 6, but the present invention is not limited to this example.

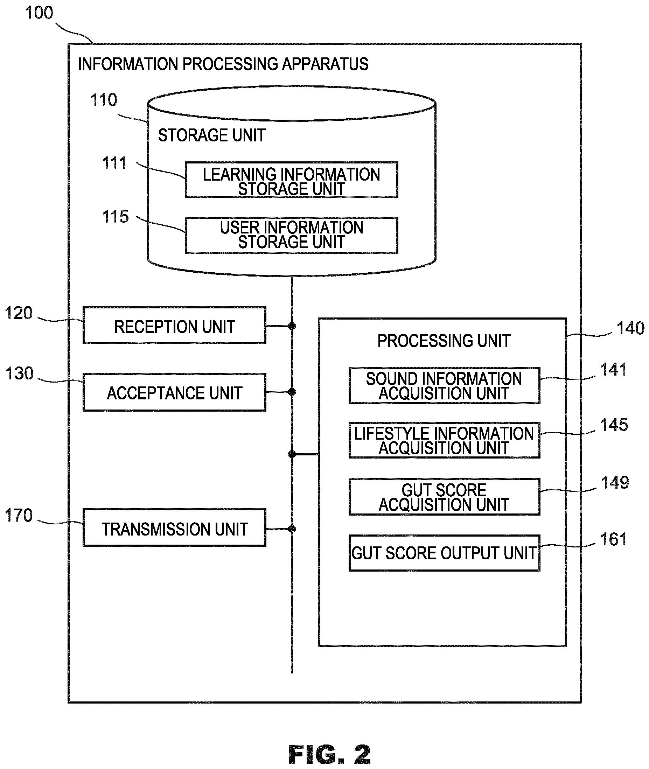

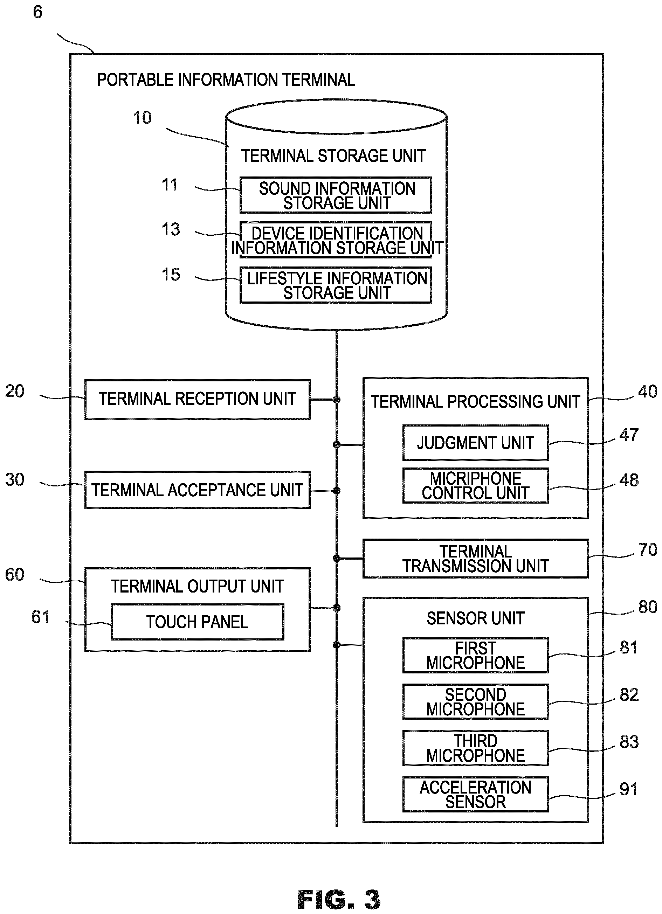

FIG. 2 is a block diagram for the information processing apparatus 100 according to the same. FIG. 3 is a block diagram for the portable information terminal 6 according to the same.

As shown in FIG. 2, the information processing apparatus 100 includes a storage unit 110, a reception unit 120, an acceptance unit 130, a processing unit 140, and a transmission unit 170. The information processing apparatus 100 is, for example, a server apparatus.

The storage unit 110 includes a learning information storage unit 111 and a user information storage unit 115.

The learning information storage unit 111 stores learning information acquired in advance. In the present embodiment, learning information is created by setting learning input information including sound information as input information, and predetermined output index values related to gut activity conditions as output information. For example, the learning information is generated by using a so-called machine learning technique as described above. For example, the learning information is generated by the information processing apparatus 100, but there is no such limitation. That is to say, the learning information generated by an apparatus other than the information processing apparatus 100 may be stored in the learning information storage unit 111.

The user information storage unit 115 stores user information. In the present embodiment, the user information is information in which user identifiers, which are identifiers that respectively identify users who use the information processing system 1, and pieces of information regarding the users are associated with each other. The user information may include various kinds of information. Examples of the user information may include information transmitted from portable information terminals 6 used by the users, information regarding the users acquired by the information processing apparatus 100 in a manner described below, and so on. Examples of the information transmitted from the portable information terminals 6 used by the users include basic information regarding the users, sound information, lifestyle information, and so on.

The reception unit 120 receives information transmitted from other apparatuses. The reception unit 120 stores the received information in the storage unit 110, for example. In the present embodiment, the users input information using the portable information terminals 6, for example, to transmit the information to the information processing apparatus 100. The reception unit 120 can store the pieces of transmitted information in the storage unit 110 in association with the user identifiers. In addition, as will be described later, in the present embodiment, sound information transmitted from each portable information terminal 6 can be received and stored in the storage unit 110 in association with a user identifier. When receiving such information from a portable information terminal 6, the reception unit 120 is able to identify the user identifier of the user related to the transmission based on the transmitted information.

The acceptance unit 130 accepts information input using an input means (not shown) connected to the information processing apparatus 100. The acceptance unit 130 stores the accepted information in the storage unit 110, for example. Note that any input means, such as a numeric keypad, a keyboard, a mouse, a menu screen, or the like, may be employed.

The processing unit 140 includes a sound information acquisition unit 141, a lifestyle information acquisition unit 145, a gut score acquisition unit 149, and a gut score output unit 161. The processing unit 140 performs various kinds of processing. The various kinds of processing are, for example, processing performed by the units included in the processing unit 140 as described below.

The sound information acquisition unit 141 acquires sound information of one user. In the present embodiment, the sound information acquisition unit 141 acquires the sound information transmitted from the user's portable information terminal 6 and received by the reception unit 120.

The lifestyle information acquisition unit 145 acquires lifestyle information. The lifestyle information acquisition unit 145 acquires information stored in the user information storage unit 115, for example. In addition, the lifestyle information acquisition unit 145 may acquire the user's basic information.

The gut score acquisition unit 149 acquires a gut score related to the user's gut condition, using input information containing the sound information acquired by the sound information acquisition unit 141 and learning information prepared in advance. In the present embodiment, the gut score acquisition unit 149 acquires predetermined output index values using the learning information stored in the learning information storage unit 111, and acquires the gut score using this output index values. For example, at least one of the bowel movement condition and the number of gut peristaltic movements per unit time is used. The number of gut peristaltic movements may be referred to as the frequency at which gut sounds at or above a predetermined level occur (hereinafter, may simply be referred to as the frequency of gut sounds).

Specifically, the gut score acquisition unit 149 acquires the gut score in the following manner, for example. The gut score acquisition unit 149 uses the sound information acquired by the sound information acquisition unit 141 and the learning information to acquire a gut condition score related to a bowel movement condition (normal, diarrhea, constipation, etc.) and a gut movement score related to gut peristaltic movements (frequency, etc.). In the present embodiment, the gut score acquisition unit 149 acquires output index values by inputting the sound information acquired by the sound information acquisition unit 141 to learning information, using a machine learning technique. Thereafter, gut-related scores (a gut condition score and a gut movement score) are acquired based on the output index values. The gut-related scores can be acquired as gut scores.

Here, in the present embodiment, the sound information input to the gut score acquisition unit 149 is a spectrum image that represents in a predetermined form the results of analyzing audio data (which may be processed) acquired by recording abdominal sounds, using the Fourier transform or the fast Fourier transform. Note that the sound information may be audio data (which may be processed) itself, or may be data converted into another format. The sound information to be input to the gut score acquisition unit 149 may be prepared in the processing unit 140 using sound information transmitted to the information processing apparatus 100 from a device that has recorded abdominal sounds, for example. Note that sound information that is a spectrum image may be prepared in advance in a device other than the information processing apparatus 100, such as a device that has recorded abdominal sounds, and the sound information may be transmitted to the information processing apparatus 100.

Note that the learning information generated for each of several types by which users can be classified by using user information may be stored in the learning information storage unit 111 in association with identifiers that identify the types. In this case, the gut score acquisition unit 149 may be configured to acquire learning information of a type corresponding to an identifier identified based on user information from the learning information storage unit 111, and acquire the gut-related scores using the learning information. This makes it possible to acquire output results with higher accuracy.

Based on information different from sound information, such as various output index values such as excretion, dietary, activity status, and various kinds of lifestyle information, the gut score acquisition unit 149 may acquire scores for the elements and may combine these scores to acquire the gut score. The scores for such elements may be acquired based on whether or not the output index values and the contents of the various kinds of lifestyle information satisfy predetermined conditions, for example. The gut score acquisition unit 149 uses the acquired gut-related scores and the scores for other elements to acquire a gut score. For example, the gut score may be acquired by calculating the gut-related scores and other scores by applying them to a predetermined calculation formula, such as by adding or multiplying them in a predetermined manner. The gut score may also be acquired using a learning model formed using a machine learning technique so as to output the gut score in response to the input scores. The scores may be normalized as necessary before being used to acquire the gut score.

The gut score acquisition unit 149 accumulates the acquired gut score in the user information storage unit 115 in association with the user identifier.

The gut score output unit 161 outputs the gut score acquired by the gut score acquisition unit 149. In the present embodiment, the gut score output unit 161 outputs information such as the gut score by transmitting it to the portable information terminal 6, but there is no such limitation. For example, the gut score output unit 161 may output the gut score by displaying it as characters or images on a display included in the information processing apparatus 100.

The transmission unit 170 transmits information to other apparatuses included in the information processing system 1 via the network. For example, the transmission unit 170 transmits information to the portable information terminal 6. In other words, for example, the transmission unit 170 outputs information to the portable information terminal 6.

Next, the configuration of the portable information terminal 6 will be described. The portable information terminal 6 is a smartphone. In the information processing system 1, the portable information terminal 6 is used as a portable information terminal for recording the user's abdominal sounds, and is also used as a terminal for outputting information regarding the gut score acquired by the information processing apparatus 100 so that the user can check it.

As shown in FIG. 3, the portable information terminal 6 includes a terminal storage unit 10, a terminal reception unit 20, a terminal acceptance unit 30, a terminal processing unit 40, a terminal output unit 60, a terminal transmission unit 70, and a sensor unit 80. The sensor unit 80 includes multiple microphones 81, 82, and 83 and an acceleration sensor 91.

The terminal storage unit 10 includes a sound information storage unit 11, a device identification information storage unit 13, and a lifestyle information storage unit 15.

The sound information storage unit 11 accumulates sound information (which may also be referred to as recorded sound information) recorded using the sensor unit 80 of the portable information terminal 6. Sound information is transmitted to the information processing apparatus 100 by the terminal transmission unit 70, and sound information may be deleted from the sound information storage unit 11 when the transmission is complete, or retained until a specified period of time has elapsed, or retained permanently until a deletion operation is performed by the user.

The device identification information storage unit 13 stores device identification information. For example, the device identification information is, for example, information such as an identifier that can identify the model (type) of the portable information terminal 6, but there is no such limitation. For example, device identification information may be an identifier that can identify the model of a device included in the portable information terminal 6 (for example, a hardware module or the like included in the microphones 81, 82, and 83). It may be possible that different information cannot be written to the device identification information storage unit 13.

The lifestyle information storage unit 15 accumulates lifestyle information. Lifestyle information is transmitted to the information processing apparatus 100 by the terminal transmission unit 70, and lifestyle information may be deleted from the lifestyle information storage unit 15 when the transmission is complete, or retained until a specified period of time has elapsed, or retained permanently until a deletion operation is performed by the user.

The lifestyle information stored in the lifestyle information storage unit 15 may include information input by the user or information based on the same. These pieces of information may be, for example, information accepted by the terminal acceptance unit 30, or information acquired by the terminal processing unit 40 by performing calculations or the like based on the information accepted by the terminal acceptance unit 30.

The lifestyle information stored in the lifestyle information storage unit 15 may include a measurement value or information based on the same. For example, these pieces of information may be information measured by the sensor unit 80, or information acquired by the terminal processing unit 40 by performing calculations, etc. based on the information measured by the sensor unit 80. These pieces of information may also be information measured by a sensor apparatus communicatively connected to the portable information terminal 6 and transmitted to the portable information terminal 6.

The terminal reception unit 20 receives information transmitted from the information processing apparatus 100 and other apparatuses via the network. The terminal reception unit 20 accumulates the received information, for example, in the terminal storage unit 10 so that the terminal processing unit 40 and so on can acquire it.

The terminal acceptance unit 30 accepts various operations input to the portable information terminal 6 by the user who uses the portable information terminal 6. For example, the operations are performed using a touch panel 61 or other operation buttons, but there is no such limitation. For example, the terminal acceptance unit 30 may accept an input operation by voice input through the microphones 81, 82, and 83. The terminal acceptance unit 30 may be regarded as accepting the information received by the terminal reception unit 20 as information input to the portable information terminal 6. That is to say, input of information to the portable information terminal 6 may be interpreted as meaning that information is indirectly input to the portable information terminal 6 by the user via another apparatus. In addition, it may be considered as inputting information to the portable information terminal 6 when the user allows information to be given to the portable information terminal 6 by, for example, executing a program that automatically generates information or giving various kinds of information to the program to make it function.

The terminal processing unit 40 performs various kinds of processing. Examples of the various kinds of processing include processing performed by the units included in the processing unit 140 using the units included in the portable information terminal 6 as appropriate, as described below.

In the present embodiment, the terminal processing unit 40 includes a judgment unit 47 and a microphone control unit 48 that perform predetermined functions when the user's abdominal sounds is to be recorded.

The judgment unit 47 judges whether or not a predetermined control condition related to recording of abdominal sounds is satisfied.

In the present embodiment, the predetermined control condition may be, for example, that the portable information terminal 6 enters a predetermined preparation state for recording abdominal sounds. For example, a predetermined preparation state for recording abdominal sounds may be, but is not limited to, the fact that a predetermined application for recording abdominal sounds is launched, the fact that a predetermined flag related to recording abdominal sounds is turned on, or the fact that a predetermined instruction from the user for recording abdominal sounds is accepted. The fact that the the portable information terminal 6 enters a predetermined preparation state for recording abdominal sounds may be understood as the fact that the the portable information terminal enters a predetermined state before starting to record abdominal sounds.

Note that, for example, the predetermined flag regarding the recording of abdominal sounds may be, but is not limited to, a flag indicating that the time for starting the predetermined recording has arrived, or a flag indicating a condition related to the surrounding environment (the volume of the ambient sound is lower than a predetermined volume, or the orientation of the portable information terminal 6 has been detected to be a predetermined orientation).

In addition, for example, the fact that a predetermined instruction is accepted from the user regarding the recording of abdominal sounds specifically refers to, but is not limited to, the acceptance of an operation to start recording or an operation to start preparation for recording.

In addition, in the present embodiment, the predetermined control condition may be, for example, that a predetermined preparation state is reached in the portable information terminal 6 during the recording abdominal sounds. For example, the fact that the predetermined state is reached during the recording of abdominal sounds may be, but is not limited to, the fact that a predetermined instruction from the user regarding recording abdominal sounds is accepted, or the fact that sound information acquired through sound recording during the recording of abdominal sounds satisfies a predetermined interruption condition.

In addition, for example, the acceptance of a predetermined instruction from the user regarding the recording of abdominal sounds specifically refers to, but is not limited to, the acceptance of an operation to interrupt recording.

In addition, for example, the fact that sound information satisfies a predetermined interruption condition may be, but is not limited to, the fact that the noise level contained in the sound information exceeds a predetermined threshold value, the fact that the signal-to-noise ratio (ratio between the noise level and the gut sound level) falls below a predetermined threshold value, or the fact that the orientation of the portable information terminal 6 is detected to be in a predetermined orientation.

Note that examples of noise may include, as described below, sounds (ambient noise) generated around the portable information terminal 6 recorded by the microphones 81, 82, 83, and so on, electromagnetic noise, and sounds (which may also be referred to as ambient noise) generated due to changes in the contact state between the portable information terminal 6 and other objects (rubbing, contact and separation, etc.). Note that the terminal processing unit 40 is configured to be able to specify noise in the sound information by a predetermined method as described below. That is to say, the terminal processing unit 40 is configured to specify noise in the sound information based on, for example, the regularity of sounds. For example, it is possible to detect whether or not the sounds of interest are sounds with regularity, and to determine whether or not the sounds are noise based on the result. The detection of whether or not sounds have regularity may be performed, for example, based on the result of the detection of the intervals between the sounds, or may be performed based on the result of the Fourier analysis on the sound information. In general, gut sounds in abdominal sounds often occur without regularity, so by specifying sounds that have a regularity as noise, gut sounds can be effectively distinguished from noise in the sound information. Note that the method of specifying noise in the sound information is not limited to this example, and various methods can be used. For example, sounds in a predetermined frequency band may be regarded as noise, or noise may be specified using learning information that can extract noise from sound information. The terminal processing unit 40 may be configured to use a combination of these methods. Note that the sound information may be transmitted to another apparatus (for example, the information processing apparatus 100 or the like) as appropriate, and specific processing related to specifying noise may be performed by the other apparatus. In this case, the result of the processing by the other apparatus may be received by the terminal reception unit 20, and the terminal processing unit 40 may be configured to specify noise based on the received result of the processing, or to perform the processing by the judgment unit 47 based on the received result of the noise specification.

The microphone control unit 48 controls one or more of the two or more microphones 81, 82, and 83 when the judgment unit 47 judges that the predetermined control condition is satisfied.

The control of the microphones 81, 82, and 83 may be, for example, on/off control that switches whether or not audio input to the microphones 81, 82, and 83 is enabled. The on/off control may be performed for each of the microphones 81, 82, and 83, but may also be performed for each group including two or more microphones. The on/off control on the microphones 81, 82, and 83 may be said to be control that disables some of the microphones 81, 82, and 83. The on/off control on the microphones 81, 82, and 83 may be said to be control for performing initialization processing on some of the microphones 81, 82, and 83 so that voice can be input thereto.

In addition, the control of the microphones 81, 82, and 83 may be the control of parameters related to the operation of the microphones 81, 82, and 83. Control of parameters includes, but is not limited to, setting, changing, deleting, etc. of parameters. Examples of parameters include a setting value that determines the gain. That is to say, the control of the microphones 81, 82, and 83 can be said to be the gain adjustment of the microphones 81, 82, and 83. Examples of the parameters may also include a value that sets whether an automatic gain adjustment function (auto gain control function) that the terminal processing unit 40 is configured to perform is enabled or disabled. That is to say, the control of the microphones 81, 82, and 83 can be said to be processing performed to enable or disable the auto gain control of at least one of the microphones 81, 82, and 83. Examples of parameters may also include parameters for processing sounds detected by the microphones 81, 82, and 83, such as parameters for turning a filter on or off for sounds in a predetermined frequency band, or parameters for adjusting the amplification of sounds in a predetermined frequency band.

In the present embodiment, for example, when it is judged that a predetermined control condition is satisfied, the microphone control unit 48 performs the following processing as a predetermined control. That is to say, the microphone control unit 48 performs control to turn on only one of the multiple microphones 81, 82, and 83 to record abdominal sounds, and turn off the other microphones. In addition, the microphone control unit 48 turns off the auto gain control function for one of the microphones used for sound recording so that the signal-to-noise ratio between the gut sounds and noise is increased. The predetermined control is not limited to this example, and other types of control may be performed. That is to say, when it is judged that a predetermined control condition is satisfied, the microphone control unit 48 may control one or more of the multiple microphones 81, 82, and 83 so that abdominal sounds are appropriately recorded.

In addition, the microphone control unit 48 may acquire device identification information, such as an identifier that identifies the model of the portable information terminal 6, from the device identification information storage unit 13, and perform a predetermined control related to the microphones 81, 82, and 83 according to the acquired identifier. For example, when the positions and numbers of microphones 81, 82, 83, etc. are different depending on the model, the microphone control unit 48 can perform a predetermined control in a manner preset for each model. In this way, it becomes possible to record high-quality abdominal sounds for each model of portable information terminal 6.

The terminal output unit 60 includes, for example, the touch panel 61, which is a display device. For example, the terminal output unit 60 outputs information by displaying the information on the touch panel 61. The method of outputting information is not limited to this example, and may be performed by outputting voice or the like from a speaker or the like.

The touch panel 61 functions as a display that displays information, and also accepts operations by the user. The touch panel 61 may be understood to be a part of the terminal acceptance unit 30. Note that a display device with only the function of displaying information may be used instead of the touch panel 61.

The terminal transmission unit 70 transmits information acquired by, for example, the terminal processing unit 40 via the network.

In the present embodiment, using the terminal transmission unit 70, the terminal output unit 60 can output sound information acquired by recording the user's abdominal sounds to the information processing apparatus 100 under the control of the terminal processing unit 40.

The sensor unit 80 records sounds using the multiple microphones 81, 82, and 83 and measures items to be measured, and outputs information such as the acquired audio data and the measurement values. The acquired information is accumulated in the terminal storage unit 10, for example. Here, examples of the measured values include a value indicating a change in acceleration obtained by the acceleration sensor, a value indicating a change in air pressure obtained by an air pressure sensor, and so on, but are not limited to these examples.

In the present embodiment, the sensor unit 80 includes three microphones, namely the first microphone 81, the second microphone 82, and the third microphone 83.

Each of the microphones 81, 82, and 83 is configured to be able to detect sounds outside the portable information terminal 6. The terminal processing unit 40 is configured to use the sounds detected by the microphones 81, 82, and 83 to record the sounds, detect a predetermined condition, accept instructions from the user, and so on. The portable information terminal 6 is configured to use such multiple microphones 81, 82, and 83 to perform various controls and information processing so as to be able to record sounds, detect sounds, and so on, with directionality and sensitivity suited to specific applications. The positions or the like of the microphones 81, 82, and 83 in the present embodiment will be described in detail later, but the specific configuration or the like is not limited thereto.

The acceleration sensor 91 is provided to detect the acceleration of the portable information terminal 6. The terminal processing unit 40 can detect the acceleration of the portable information terminal 6 using the acceleration sensor 91. That is to say, the terminal processing unit 40 can detect the orientation of the portable information terminal 6 when the portable information terminal 6 is substantially stationary.

Note that the sensor unit 80 may include, for example, a pulse sensor, an illuminance sensor, a camera, and a position information sensor that can specify a position using a GPS or the like. The sensor unit 80 may also be a timer that measures the passage of time. The portable information terminal 6 may be configured to function as an activity meter for acquiring information regarding the user's lifestyle habits, such as information regarding the user's activity level, such as calories burned and number of steps taken, the user's wake-up time, bedtime, and commute time, and so on. Note that information regarding the user's living environment, such as the area in which the user lives, the climate, the noise environment, etc., may be acquired based on the measurement values acquired by the sensor unit 80 and accumulated in the terminal storage unit 10.

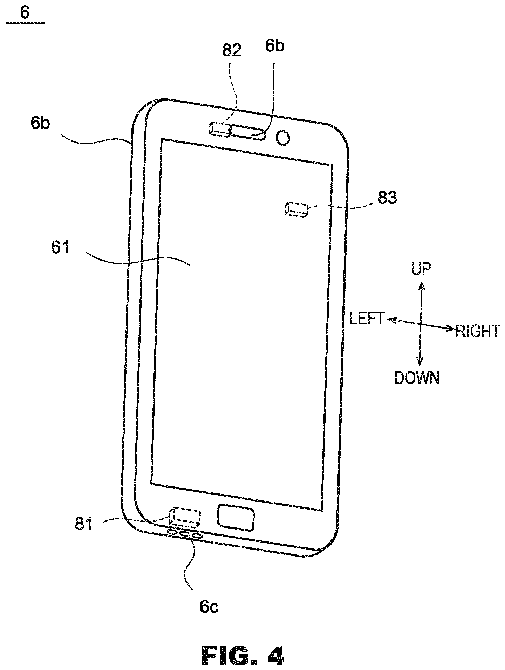

FIG. 4 is a perspective view showing the portable information terminal 6 according to the same.

In the present embodiment, the portable information terminal 6 has a housing 6b having a rectangular plate shape. One surface (hereinafter referred to as a front surface) of the housing 6b is provided with the touch panel 61. The microphones 81, 82, and 83 are accommodated in the housing 6b.

In the present embodiment, the first microphone 81 is disposed near an end portion of the housing 6b. The portion of the housing 6b near the first microphone 81 is a sound collecting portion 6c. The sound collecting portion 6c is, for example, an opening formed in the housing 6b so as to enable transmission of sound from the outside of the housing 6b to the first microphone 81, but is not limited to this example.

In the present embodiment, the sound collecting portion 6c is disposed at an end portion of the housing 6b. It can be said that, when the surface on which the touch panel 61 is arranged is defined as the upper surface, the sound collecting portion 6c is disposed on a side edge portion of the housing 6b. The sound collecting portion 6c may also be said to be arranged on the side peripheral portion of the housing 6b.

More specifically, the sound collecting portion 6c is located at the bottom of the portable information terminal 6. The sound collecting portion 6c may also be said to be arranged at the lower edge of the housing 6b. The portable information terminal 6 is provided with an output port 6d for a speaker for calls at a top portion thereof. When the portable information terminal 6 is held close to the user's head for a call, the output port 6d is located at an upper portion close to the user's ear, and the sound collecting portion 6c is located at a lower portion close to the user's mouth. Note that, in the present embodiment, the term "lower" refers to the lower side of the display screen, contents, and the like that are displayed on the touch panel 61 when the portable information terminal 6 is used in a normal manner.

The second microphone 82 is disposed near an upper portion of the housing 6b. For example, the second microphone 82 is disposed near the output port 6d. For example, the second microphone 82 is configured to be able to detect sound entering the inside of the housing 6b from the front side of the housing 6b via the output port 6d.

For example, the third microphone 83 is disposed near the rear surface of the housing 6b. The third microphone 83 is configured to be able to detect sound entering the inside of the housing 6b from the rear side of the housing 6b. Note that an opening may be provided in the housing 6b corresponding to the third microphone 83 so that sound enters the third microphone 83.

These microphones 81, 82, and 83 are controlled by the terminal processing unit 40 so as to fulfill their respective roles according to the operation mode of the portable information terminal 6. For example, in the present embodiment, during a call, for example, the first microphone 81 is mainly used as a microphone for inputting the voice uttered by the user. In this case, the second microphone 82 and the third microphone 83 can be used to detect the surrounding ambient noise and perform processing to cancel out sounds detected by the first microphone 81 other than sounds emitted by the user. In addition, when capturing moving images or the like using the portable information terminal 6, an appropriate one of the microphones 81, 82, and 83 can be used so as to effectively record sounds from the range captured by a camera (not shown).

In the present embodiment, the first microphone 81 is used to record the user's abdominal sounds. The recording of abdominal sounds using the portable information terminal 6 can be performed in the manner described below, for example.

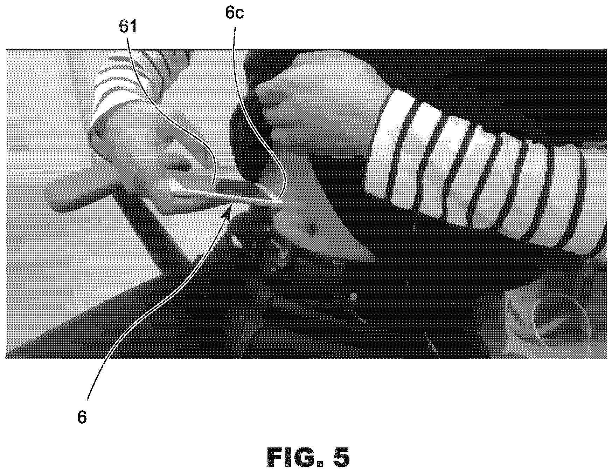

FIG. 5 is a diagram showing an example of a situation in which a user records abdominal sounds with the portable information terminal 6 according to the same.

As shown in FIG. 5, in the present embodiment, the user's abdominal sounds are recorded by bringing the sound collecting portion 6c of the housing 6b near the first microphone 81 into contact with the user's abdomen. In this case, it is preferable that the user is in a sitting position, such as on a chair or the like, and in a resting state. It is preferable that the sound collecting portion 6c is placed in direct contact with the skin of the abdomen. For adult users, the contact site is preferably located 9 cm to the left or right of the navel. In the present embodiment, the user starts sound recording while gently pressing the sound collecting portion 6c against the abdomen. The abdominal sound recording will stop after 60 seconds of recording. Note that, in order to acquire a higher-quality recording result, it is preferable that the touch panel 61, i.e., the portable information terminal 6, is held in a position such that it is approximately perpendicular to the plane that is in contact with the surface of the abdomen at the contact point of the sound collecting portion 6c with the abdomen.

In the present embodiment, when the user's abdominal sounds are recorded by the portable information terminal 6 in this manner, the judgment unit 47 and the microphone control unit 48 control the microphones 81, 82, and 83 in the following manner, for example.

That is to say, the judgment unit 47 judges that the predetermined control condition is satisfied if an operation to start the recording of abdominal sounds has been performed. That is to say, the predetermined control condition is, for example, that an operation to start the recording of abdominal sounds has been performed.

In addition, when the judgment unit 47 judges that the predetermined control condition is satisfied, i.e., if an operation to start the recording of abdominal sounds has been performed, the microphone control unit 48 turns on the first microphone 81 and sets the auto gain control function for the first microphone 81 to off. In other words, the microphone control unit 48 performs initialization processing for recording abdominal sounds with the first microphone 81. On the other hand, the microphone control unit 48 turns off the second microphone 82 and the third microphone 83, which are not used for recording abdominal sounds. In other words, the microphone control unit 48 performs control to disable the microphones that are not used for recording among the multiple microphones 81, 82, and 83. As a result, abdominal sounds are recorded using only the first microphone 81, and sound information is acquired.

Note that, regardless of such control, the other microphones 82 and 83 may also be used to record abdominal sounds in addition to the first microphone 81. For example, the microphone control unit 48 may perform processing to set the gain of each of the three microphones 81, 82, and 83 to a predetermined setting value suitable for recording abdominal sounds. The microphones 82 and 83 may be controlled so that information for canceling out the ambient noise is acquired from the recording result of the first microphone 81 by using at least one of the microphones 82 and 83.

Note that, in the present embodiment, the terminal processing unit 40 may be configured to, when the user's abdominal sounds are to be recorded, detect the environment in which the sound recording is to be performed and to perform processing related to the sound recording in accordance with the detection result. For example, the terminal processing unit 40 may perform detection with respect to the environment in which the sound recording is performed, based on sound information acquired by the sound recording performed using the first microphone 81. The sound information used at this time may be sound information recorded for the purpose of recording abdominal sounds, or a recording of sounds collected by the first microphone 81 prior to recording the abdominal sounds. In addition, the environment in which recording is performed may be detected based on sound information acquired using the second microphone 82 and the third microphone 83 that are not used to record abdominal sounds.

More specifically, for example, the terminal processing unit 40 may detect whether or not the environment allows appropriate recording based on on the noise level in the sound information and the signal-to-noise ratio between abdominal sounds and noise. The terminal processing unit 40 may perform control so that sound recording can be performed when the environment is suitable for appropriate sound recording, or may perform control so that sound recording cannot be performed when the environment is not suitable for appropriate sound recording. If the environment is not suitable for appropriate sound recording, the terminal processing unit 40 may notify the user so that the user can improve the environment.

By performing such processing, it is possible to acquire a higher-quality recording result. Note that such processing may be performed according to the judgment result of the judgment unit 47 or the control of the microphone control unit 48, or may be performed regardless of the judgment result of the judgment unit 47 or the control of the microphone control unit 48.

Next, examples of operations of the portable information terminal 6 and examples of operations of the information processing apparatus 100 performed when a user uses the information processing system 1 according to the present embodiment will be described. In the present embodiment, a user can use the information processing system 1, for example, by accessing the information processing apparatus 100 and receiving information transmitted from the information processing apparatus 100 using the portable information terminal 6, and by running a predetermined application on the portable information terminal 6. Note that the predetermined application may be, for example, a dedicated application that runs using information transmitted from the information processing apparatus 100, or a web browser or the like that displays web applications provided in the information processing apparatus 100 so that the user can use the applications.

In the present embodiment, the information processing system 1 is typically used in the following manner. That is to say, the user uses the portable information terminal 6 to record their own abdominal sounds. The sound information is transmitted from the portable information terminal 6 to the information processing apparatus 100, and the gut score is acquired in the information processing apparatus 100. The information processing apparatus 100 transmits (outputs) the acquired gut score to the portable information terminal 6. The portable information terminal 6 receives the gut score and displays information including the gut score on the display device via the terminal output unit 60. As a result, the user can confirm information regarding their own intestines as a gut score. When such operations of the information processing system 1 are performed, the portable information terminal 6 and the information processing apparatus 100 perform various operations in the following manner, for example. These operations are performed by the terminal processing unit 40 and the processing unit 140 performing control operations or the like using each unit.

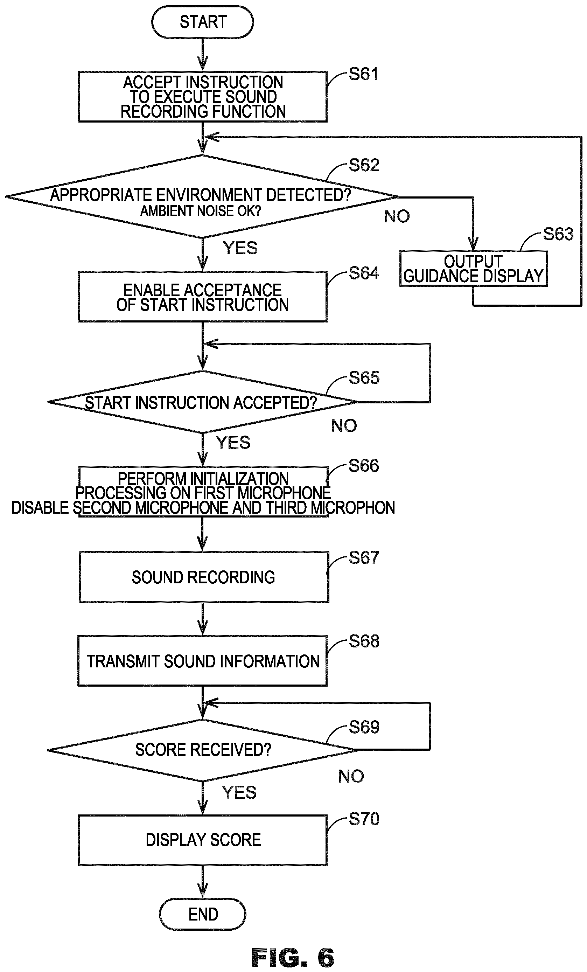

FIG. 6 is a flowchart showing examples of operations of the information processing apparatus 100 according to the same.

(Step S61) The terminal processing unit 40 receives an instruction to execute the sound recording function of recording abdominal sounds. For example, when an instruction to start a predetermined application or an operation to select a predetermined operation mode is performed, the terminal processing unit 40 accepts an instruction to execute the sound recording function.

To perform sound recording, the user takes a sitting position and presses the sound collecting portion 6c of the portable information terminal 6 against their abdomen.

(Step S62) The terminal processing unit 40 judges whether or not the environment is an appropriate environment in which appropriate sound recording can be performed, i.e., whether or not it is detected that the environment is an appropriate environment. Here, for example, it is detected that the environment is an appropriate environment when the fact (the predetermined condition) that the volume of the ambient noise is smaller than a predetermined value is satisfied. If it is detected that the environment is an appropriate environment, processing proceeds to step S64, and otherwise processing proceeds to step S63.

Note that the predetermined condition for detecting that the environment is an appropriate environment is not limited to this example, and a condition such as the condition that the portable information terminal 6 is in a substantially stopped state or the condition that the portable information terminal 6 is in a predetermined orientation may also be used. Also, the environment may be detected as an appropriate environment when all of multiple conditions are satisfied.

(Step S63) The terminal processing unit 40 outputs a guidance display indicating that the environment is not an appropriate environment. For example, the information is displayed on the touch panel 61. Thereafter, processing returns to step S62. Note that if such processing is not performed and it is not detected in step S62 that the environment is an appropriate environment, processing may be held in standby.

(Step S64) The terminal processing unit 40 controls the portable information terminal 6 so that the portable information terminal 6 can accept a start instruction from the user. For example, an operation button for starting recording of abdominal sounds (for starting recording of abdominal sounds as sound information) is enabled on the operation interface displayed on the touch panel 61. For example, the display state may be changed, such as by making the button appear or canceling the grayed-out state, or the behavior when the operation button is operated may be changed.

(Step S65) The terminal processing unit 40 judges whether or not a start instruction has been accepted from the user. The terminal processing unit 40 waits until a start instruction is accepted, and processing proceeds to step S66 when the start instruction is accepted. When the start instruction is accepted, the terminal processing unit 40 judges that the predetermined control condition is satisfied.

(Step S66) The terminal processing unit 40 controls the multiple microphones 81, 82, and 83 so that abdominal sounds are appropriately recorded. In the present embodiment, as described above, the terminal processing unit 40 turns on the first microphone 81 and sets the auto gain control function for the first microphone 81 to off (initialization processing). In addition, the microphone control unit 48 turns off (disables) the second microphone 82 and the third microphone 83 that are not used for recording abdominal sounds.

(Step S67) The terminal processing unit 40 performs sound recording. Sound recording is performed using only the first microphone 81, which is turned on. Note that sound recording is performed for a predetermined period of time required to acquire a score (for example, 60 seconds). As a result, a recording result of abdominal sounds is acquired.

While sound recording are being performed as described above, the terminal processing unit 40 may be configured to output a display related to the sound recording status on the touch panel 61 so that the user can visually check it. The display related to the sound recording status may be, for example, a display showing the progress status (for example, a display showing the remaining time, a progress bar, or the like).. The sound recording status display can be a waveform or the like that shows the contents of the recorded sound information. Providing such a display helps the user maintain the stopped state necessary to record high-quality abdominal sounds.

(Step S68) The terminal processing unit 40 transmits the sound information to the information processing apparatus 100. Note that if the sound information cannot be transmitted properly due to a communication problem or the like, a display may be displayed suggesting to the user to move to another location or change the communication means.

As a result, when the sound information is transmitted to the information processing apparatus 100, the sound information is used in the information processing apparatus 100 to acquire a gut score, and information regarding the acquire gut score or the like is transmitted from the portable information terminal 6 to the information processing apparatus 100.

(Step S69) The terminal processing unit 40 judges whether or not information regarding the score transmitted from the information processing apparatus 100 has been received. The terminal processing unit 40 waits until the information is received, and processing proceeds to step S70 when the start instruction is received.

(Step S70) The terminal processing unit 40 uses the received information regarding the score to output a display related to the gut score or the like on the touch panel 61. As a result, the user can know their own gut score or related information. Thereafter, the series of processing is terminated.

Note that, based on the results of the sound recording, if there is an abnormality such as the abdominal sounds do not contain gut sounds or there are more than a certain volume of sounds judged to be gut sounds, the user may be notified of this abnormality or be suggested to perform the sound recording again. The presence or absence of such an abnormality may be judged within the portable information terminal 6 based on the sound information acquired, or the sound information may be transmitted to the information processing apparatus 100 and the result of the judgment performed by the information processing apparatus 100 may be fed back to the portable information terminal 6.

In addition, the detection of an appropriate environment and the processing related thereto (steps S62 and S63) may not be performed. In addition, the processing in step S66 may be performed when an instruction to execute a sound recording function is accepted (an example of a predetermined control condition). That is to say, the processing may be performed after step S61.

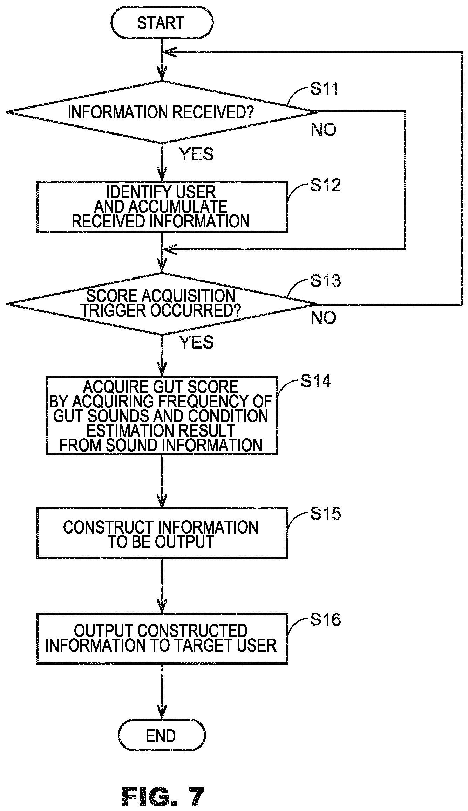

FIG. 7 is a flowchart showing examples of operations of the information processing apparatus 100 according to the same.

(Step S11) The processing unit 140 judges whether or not the reception unit 120 has received information transmitted from the portable information terminal 6 or the like. If it is judged that information has been received, processing proceeds to step S12, and otherwise processing proceeds to step S13.

(Step S12) The processing unit 140 identifies the user based on the received information, and accumulates the received information in the user information storage unit 115 in association with the user identifier. For example, when lifestyle information, sound information, device identification information, or the like is transmitted from the portable information terminal 6 in association with the user identifier, the processing unit 140 accumulates the received information in the user information storage unit 115 in association with the user identifier.

(Step S13) The processing unit 140 judges whether or not a trigger for acquiring a gut score has occurred. In other words, the processing unit 140 judges whether or not the conditions for starting to acquire a gut score are satisfied. If it is judged that a trigger has occurred, processing proceeds to step S14. Otherwise, processing returns to step S11.

Note that, for example, the above trigger can be an instruction from the user via the portable information terminal 6 to acquire a gut score (the transmission of predetermined information corresponding to the instruction), but the trigger is not limited to this example. For example, the trigger can be the satisfaction of various conditions, such as reaching a predetermined point in time, receiving new sound information or other lifestyle information, and so on.

(Step S14) The processing unit 140 performs score acquisition processing, using the gut score acquisition unit 149. Score acquisition processing will be described later. A gut score and each element score are acquired and accumulated in the user information storage unit 115 through score acquisition processing.

(Step S15) The processing unit 140 causes the gut score output unit 161 to construct information to be output, using the acquired gut score and so on. In the present embodiment, information for showing the gut score and information for showing the element scores as a radar chart are constructed.

(Step S16) The processing unit 140 causes the gut score output unit 161 to output the constructed information to the target user. That is to say, the processing unit 140 outputs, via the transmission unit 170, the information constructed with respect to the gut score and the element scores to the portable information terminal 6 used by the user for whom the score is calculated. As a result, it is possible to display the gut score and the element scores on the portable information terminal 6 that has received the information.

Next, specific examples of the sound recording of abdominal sounds according to the present embodiment will be described while showing screen transitions on the portable information terminal 6 used by the user.

In the following specific examples, it is assumed that the information processing system 1 provides a health support application to assist the user in leading a healthy life. The health support application accepts input operations related to the user's lifestyle information and provides the user with information useful for maintaining health. Th health support application is realized by the user executing a predetermined application on the portable information terminal 6, and the portable information terminal 6 and the information processing apparatus 100 performing communication with each other. The example screens of the health support application described below are displayed by the terminal output unit 60 under the control of the terminal processing unit 40.

While the health support application is running, the user can record their own abdominal sounds to acquire a gut score, by performing a predetermined operation.

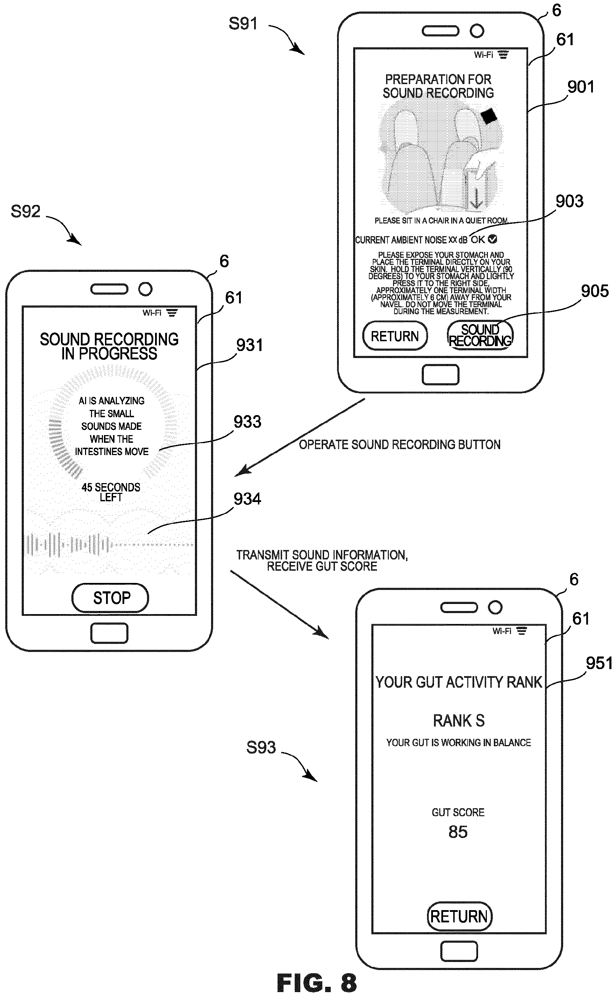

FIG. 8 is a diagram showing specific examples of screen transitions of the portable information terminal 6 according to the same.

In FIG. 8, in step S91, a sound recording preparation screen 901 for performing sound recording using the health support application is displayed. The sound recording preparation screen 901 includes, for example, a display guiding the user to sit down and press the portable information terminal 6 against their abdomen in order to record sounds. The user makes the necessary preparations according to the displayed contents of the recording preparation screen 901, and the user can start sound recording by operating a sound recording button 905.

In the example shown in the figure, the sound recording preparation screen 901 includes an environment information display 903 that shows the result of measuring the ambient noise and the result of detecting that the environment is an appropriate environment. As a result, the user can easily know information regarding ambient noise, such as whether or not the environment is an appropriate environment and the volume of the ambient noise. The display mode of the environment information display 903 may be changed depending on whether the environment is detected as an appropriate environment or not. Also, the configuration may be such that the operation on the sound recording button 905 can be accepted only when the environment is detected as an appropriate environment.

When the sound recording button 905 is operated, the judgment unit 47 detects that the control condition is satisfied, and the microphone control unit 48 controls the microphones 81, 82, and 83 to perform sound recording. Thereafter, the recording of abdominal sounds is started.

The user can start recording abdominal sounds by to operating the sound recording button 905. Using only the first microphone 81 used for recording abdominal sounds, recording is performed under conditions suitable for recording abdominal sounds. Therefore, it is possible to acquire a high-quality recording result. In this example, the recording of abdominal sounds starts in the state where the environment has been detected as an appropriate environment, and therefore it is possible to acquire an even higher quality recording result of abdominal sounds.

When the sound recording button 905 is operated, the screen transitions to a recording-in-progress screen 931 (step S92). The recording-in-progress screen 931 shows a recording progress display 933 and a recording waveform display 934 as displays related to the sound recording status. The recording progress display 933 may be, for example, a remaining time indicator or a progress bar indicating the degree of progress, but is not limited to this example. The recording waveform display 934 displays a waveform indicating the contents of the recorded sound information and allows the user to have a realistic sense that sound recording is in progress.

In the example shown in the figure, the recording-in-progress screen 931 includes such display of the sound recording status, and therefore it is possible to attract the user's attention during the sound recording and prevent the user from making unnecessary movements that are one of the factors that may reduce the quality of the sound recording. In addition, the user can check the progress, and therefore the user can wait for the sound recording to be completed without worrying.

If the sound recording is performed normally, the sound information is transmitted to the information processing apparatus 100, and the gut score is transmitted from the information processing apparatus 100 to the portable information terminal 6. When the portable information terminal 6 receives the gut score, the screen transitions to a result display screen 961 (step S93). The result display screen 951 displays, for example, the acquired gut score and information regarding the score (for example, information indicating a rank classified according to the degree of gut activity, etc.). The user can check the contents of the result display screen 951, recognize an objective evaluation of the condition of their own intestines, and use this information in their daily life.

As described above, in the present embodiment, abdominal sounds are recorded using the portable information terminal 6 after controlling the multiple microphones 81, 82, and 83 to use a microphone suitable for sound recording. Therefore, a high-quality recording result of abdominal sounds can be acquired. In addition, the processing related to sound recording is performed based on the result of the detection that the environment is an appropriate environment, and therefore a high-quality recording result of abdominal sounds can be acquired. When the environment is not an appropriate environment, a corresponding display is displayed on the touch panel 61, thereby urging the user to record sounds in a more appropriate environment. Note that a high-quality recording result specifically means, but is not limited to, a high signal-to-noise ratio, and can mean, for example, a high level of abdominal sounds, a low level of noise, or other conditions in which abdominal sounds (especially gut sounds) can be more clearly heard.

The information processing apparatus 100 uses high-quality sound information to acquire a gut score related to the user's gut condition, and outputs the acquired gut score, and therefore the information processing apparatus 100 can output a gut score that accurately represents the user's gut condition. Since such a gut score is output, the user can easily know objective information regarding the condition of their own intestines, which can be easily grasped.

It is preferable that the above storage unit 110 and the terminal storage unit 10 are realized using a non-volatile recording medium, but they can be realized using a volatile recording medium. Information or the like acquired by the apparatuses is stored in the storage units, but there is no limitation on the process in which information or the like is stored therein. For example, information or the like may be stored via a recording medium, or information or the like transmitted via a communication line or the like may be stored, or information or the like input via an input device may be stored.

The above processing unit 140 and the terminal processing unit 40 are typically realized using an MPU, a memory, or the like. The processing procedures performed by the processing unit 140 and the terminal processing unit 40 are typically realized using software, and the software is recorded on a recording medium such as a ROM. However, such processing procedures may be realized using hardware (a dedicated circuit).

In addition, the input means that can be used to input information that can be accepted by the acceptance unit 130 and the terminal acceptance unit 30 may be any means, such as a numeric keypad, a keyboard, a mouse, or a menu screen. The acceptance unit 130 and the terminal acceptance unit 30 can be realized by a device driver for an input means such as a numeric keypad or a keyboard, or control software for a menu screen.

In addition, the reception unit 120 and the terminal reception unit 20 are typically realized using a wireless or wired communication means, but may be realized using a broadcast reception means.

In addition, the transmission unit 170 and the terminal transmission unit 70 are typically realized using a wireless or wired communication means, but may be realized using a broadcast means.

Note that the information processing apparatus 100 may be constituted by a single server, or may be constituted by multiple servers operating in cooperation with each other, or may be a computer or the like built into other devices. Note that the server may be a so-called cloud server, an ASP server, or the like, and of course the type thereof is not limited.

In addition, some or all of the units provided in the information processing apparatus 100 described above may be provided in the portable information terminal 6. That is to say, the portable information terminal 6 may have a part or all of the configuration for realizing the functions related to acquiring and outputting the gut score, such as those of the information processing apparatus 100 described above. For example, the portable information terminal 6 may be configured to be able to acquire the gut score, using sound information recorded by the portable information terminal 6. On the other hand, some of the units provided in the portable information terminal 6 described above may be provided in the information processing apparatus 100. That is to say, the information processing apparatus 100 may include a part of the configuration for detecting whether or not the environment is an appropriate environment as the portable information terminal 6 described above includes. For example, the information processing apparatus 100 may be configured to detect an appropriate environment using the sound information recorded by the portable information terminal 6, and transmit a sound recording start instruction to the portable information terminal 6 based on the result of the detection.

The processing in the present embodiment may be realized using software. This software may be distributed through software downloading or the like. Also, this software may be recorded on a recording medium such as a CD-ROM and distributed.

Note that the software that realizes the information processing apparatus 100 in the present embodiment is the program described below. That is to say, this program is a program that enables a computer to function as: a sound information acquisition unit that acquires sound information regarding a user's abdominal sounds, which are sounds emitted from the user's abdomen; a gut score acquisition unit that acquires a gut score related to the user's gut condition, using input information containing the sound information acquired by the sound information acquisition unit and learning information prepared in advance; and an output unit that outputs the gut score acquired by the gut score acquisition unit.

The software that realizes the portable information terminal 6 in the present embodiment, which is a portable information terminal, is the program described below. That is to say, this program is a program that enables a computer of a portable information terminal that has two or more microphones and is used to record a user's abdominal sounds in a state where a sound collecting portion near one of the two or more microphones is in contact with the user's abdomen, to function as: a judgment unit that judges whether or not a predetermined control condition related to recording of the abdominal sounds is satisfied; and a microphone control unit that controls one or more microphones among the two or more microphones when the control condition is judged to be satisfied.

### Others

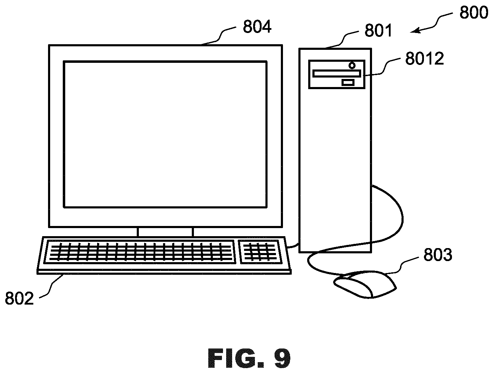

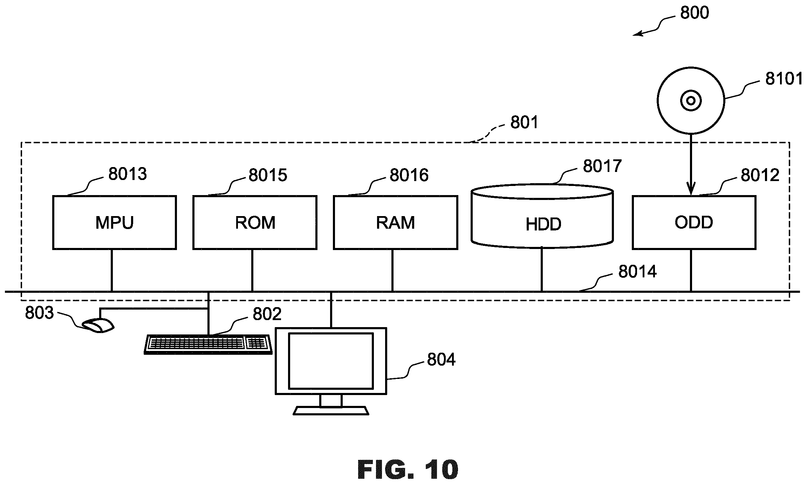

FIG. 9 is an overview diagram for a computer system 800 in the above embodiment. FIG. 10 is a block diagram for the computer system 800.

These figures show an example of the configuration of a computer that executes the program described in this specification to realize the information processing apparatus 100 and the like according to the above-described embodiments. The above-described embodiments can be realized using computer hardware and a computer program executed thereon.

The computer system 800 includes a computer 801 that includes an optical disc drive, a keyboard 802, a mouse 803, and a monitor 804.

The computer 801 includes, in addition to the optical disc drive (ODD) 8012, an MPU 8013, a bus 8014 that is connected to the optical disc drive 8012 and so on, a ROM 8015 for storing programs such as a boot-up program, a RAM 8016 that is connected to the MPU 8013 and is used to temporarily store application program instructions and provide a temporary storage space, and a hard disk (HDD) 8017 for storing application programs, system programs, and data. Here, although not shown in the figure, the computer 801 may further include a network card that provides connection to a LAN.

The program that enables the computer system 800 to perform the functions of the information processing apparatus and so on according to the above-described embodiments may be stored in the optical disc 8101, inserted into the optical disc drive 8012, and furthermore transferred to the hard disk 8017. Alternatively, the program may be transmitted to the computer 801 via a network (not shown) and stored on the hard disk 8017. The program is loaded into the RAM 8016 when the program is to be executed. The program may be loaded from the optical disc 8101 or directly from the network.

The program does not necessarily have to include an operating system (OS), a third-party program, or the like that enables the computer 801 to perform the functions of the information processing apparatus and so on according to the above-described embodiments. The program need only contain the part of the instruction that calls an appropriate function (module) in a controlled manner to achieve a desired result. How the computer system 800 works is well known and the detailed descriptions thereof will be omitted.

In the above-described program, the step of transmitting information, the step of receiving information, and so on do not include processing performed by hardware, for example, processing performed by a modem or an interface card in the step of transmitting (processing that can only be performed by hardware).

There may be a single or multiple computers executing the above-described program. That is to say, centralized processing or distributed processing may be performed.

In the above-described embodiments, two or more constituent elements that are present in one apparatus may be physically realized using one medium.

Also, in the above-described embodiments, each kind of processing (each function) may be realized as centralized processing that is performed by a single apparatus (system), or distributed processing that is performed by multiple apparatuses (in this case, the entire system constituted by multiple apparatuses performing distributed processing can be considered as a single "apparatus").

In addition, in the above-described embodiments, the transfer of information between constituent elements may be performed, for example, by one constituent element outputting information and the other constituent element receiving information if the two constituent elements transferring the information are physically different, or, by shifting from a processing phase corresponding to one constituent element to a processing phase corresponding to the other constituent element if the two constituent elements transferring the information are physically the same.

In addition, in the above-described embodiments, information related to the processing performed by each constituent element, for example information accepted, acquired, selected, generated, transmitted, or received by each constituent element, and information such as threshold values, formulas, addresses, etc. used by each constituent element in processing, may be stored temporarily or for a long period of time on a recording medium (not shown), even if not explicitly stated in the above description. In addition, the accumulation of information in a recording medium (not shown) may be performed by each constituent element or an accumulation unit (not shown). In addition, reading of information from the recording medium (not shown) may be performed by each constituent element or a reading unit (not shown).

In addition, in the above-described embodiments, if information used by each constituent element, such as threshold values, addresses, various setting values, etc. used by each constituent element in processing may be changed by the user, the user may or may not be able to change such information as appropriate, even if it is not explicitly stated in the above description. If these pieces of information can be changed by the user, the change may be realized, for example, by an acceptance unit (not shown) that accepts a change instruction from the user, and a change unit (not shown) that changes the information in accordance with the change instruction. The acceptance of the change instruction by the acceptance unit (not shown) may be, for example, acceptance from an input device, reception of information transmitted via a communication network, or acceptance of information read out from a predetermined recording medium.

The present invention is not limited to the above-described embodiments, and various modifications are possible, which are also included within the scope of the present invention.

Some of the component elements and functions of the above-described embodiments may be omitted.

### Industrial Applicability

As described above, the portable information terminal according to the present invention has the effect of being able to acquire a high-quality recording result of abdominal sounds, and is useful as a portable information terminal or the like.

### List of Reference Numerals

1 Information Processing System
6 Portable Information Terminal
10 Terminal Storage Unit
11 Sound Information Storage Unit
13 Device Identification Information Storage Unit
15 Lifestyle Information Storage Unit
20 Terminal Reception Unit
30 Terminal Acceptance Unit
40 Terminal Processing Unit
47 Judgment Unit
48 Microphone Control Unit
60 Terminal Output Unit
61 Touch Panel
70 Terminal Transmission Unit
80 Sensor Unit
81 First Microphone
82 Second Microphone
83 Third Microphone
91 Acceleration Sensor
100 Information Processing Apparatus
110 Storage Unit
111 Learning Information Storage Unit
115 User Information Storage Unit
120 Reception Unit
130 Acceptance Unit
140 Processing Unit
141 Sound Information Acquisition Unit
149 Gut score Acquisition Unit
161 Gut score Output Unit
170 Transmission Unit

## Claims

1. A portable information terminal that has two or more microphones and is used to record a user's abdominal sounds in a state where a sound collecting portion near one of the two or more microphones is in contact with the user's abdomen, the portable information terminal comprising:
a judgment unit that judges whether or not a predetermined control condition related to recording of the abdominal sounds is satisfied; and
a microphone control unit that controls one or more microphones among the two or more microphones when the control condition is judged to be satisfied.

2. The portable information terminal according to claim 1,
wherein the microphone control unit performs on and off control on the one or more microphones when the control condition is judged to be satisfied.

3. The portable information terminal according to claim 1,
wherein the microphone control unit controls parameters related to operation of the one or more microphones when the control condition is judged to be satisfied.

4. The portable information terminal according to claim 1,
wherein the microphone control unit acquires an identifier identifying a model of the portable information terminal, and controls the one or more microphones in accordance with the acquired identifier.

5. The portable information terminal according to claim 1,
wherein the predetermined control condition is that the portable information terminal enters a predetermined preparation state for recording the abdominal sounds.

6. The portable information terminal according to claim 1,
wherein the predetermined control condition is that sound information acquired through the recording satisfies a predetermined interruption condition during the recording of the abdominal sounds.

7. An information processing system comprising: the portable information terminal according to claim 1; and an information processing apparatus capable of communicating with the portable information terminal,
wherein the portable information terminal includes a terminal output unit that outputs sound information acquired by recording a user's abdominal sounds using the one or more microphones, and
the information processing apparatus includes:
a sound information acquisition unit that acquires the sound information;
a gut score acquisition unit that acquires a gut score related to the user's gut condition, using input information containing the sound information acquired by the sound information acquisition unit and learning information prepared in advance; and
a gut score output unit that outputs the gut score acquired by the gut score acquisition unit.

8. A method for controlling a portable information terminal that has two or more microphones and is used to record a user's abdominal sounds in a state where a sound collecting portion near one of the two or more microphones is in contact with the user's abdomen, the method comprising:
a judgment step of judging whether or not a predetermined control condition related to recording of the abdominal sounds is satisfied; and
a microphone control step of controlling one or more microphones among the two or more microphones when the control condition is judged to be satisfied.

9. Aprogram that enables a computer of a portable information terminal that has two or more microphones and is used to record a user's abdominal sounds in a state where a sound collecting portion near one of the two or more microphones is in contact with the user's abdomen, to function as:
a judgment unit that judges whether or not a predetermined control condition related to recording of the abdominal sounds is satisfied; and
a microphone control unit that controls one or more microphones among the two or more microphones when the control condition is judged to be satisfied.
